(19)

Europäisches
Patentamt
European
Patent Office
Office européen
des brevets

(11) **EP 2 609 858 A1**

(12) **EUROPEAN PATENT APPLICATION**

(43) Date of publication:
**03.07.2013 Bulletin 2013/27**

(51) Int Cl.:
*A61B 5/11* (2006.01)     *A61B 5/22* (2006.01)
*A63B 24/00* (2006.01)

(21) Application number: **12191069.9**

(22) Date of filing: **02.11.2012**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **28.12.2011 KR 20110144937**

(71) Applicant: **Samsung Electronics Co., Ltd
Gyeonggi-do 443-742 (KR)**

(72) Inventors:
• **Kim, Jung-hoon
Gyeonggi-do (KR)**

• **Kim, Jong-lok
Seoul (KR)**
• **Kim, Sung-soo
Seoul (KR)**
• **Choi, Jong-il
Seoul (KR)**

(74) Representative: **Hewett, Jonathan Michael
Richard et al
Venner Shipley LLP
200 Aldersgate
London EC1A 4HD (GB)**

(54)     **Method for measuring quantity of exercise and display apparatus thereof**

(57)     An exercise quantity measuring method and display apparatus thereof is provided. The exercise quantity measuring method includes photographing a user's motion to obtain a motion image, calculating a motion analogous degree representing a comparison between the motion image and a sample motion image, where the sample motion image is previously stored in association with a sample value of exercise quantity; and calculating an exercise quantity of the user's motion based on the motion analogous degree and the sample value of exercise quantity.

# FIG. 2

EP 2 609 858 A1

**Description**

The present invention relates to a method for measuring a quantity of exercise and a display apparatus therefor, and more particularly to a method for measuring a quantity of exercise in which movements of a user are photographed and a quantity of exercise of the user is measured, and a display apparatus therefor.

In modern societies, as living quality improves and the senior population increases, people's interest in health is increasing. Especially, more and more programs are being created for managing people's health and exercise using display apparatuses such as TVs.

Of health related programs, there are programs which provide health management services to users by measuring their quantity of exercise. Some programs may use methods of measuring the quantity of exercise of a user using a sensor attached to a particular body part of the user.

In the case of measuring a user's exercise quantity using a sensor (for example, an acceleration sensor etc.) attached to a particular body part of the user, it is difficult figure out overall body movements of the user, and thus it is difficult to measure an exact quantity of exercise, which is a problem. Furthermore, in such a case of attaching a sensor to a particular body part of the user, there is an inconvenience in that movements of the user may be restrained.

Therefore, there is need to seek a method of measuring a user's quantity of exercise which uses overall movements of the user without attaching a sensor to a body part of the user.

One or more exemplary embodiments of the present invention provide a method for measuring a motion analogous degree representing a comparison between of a motion image and a sample motion image stored in association with a sample value of exercise quantity, and using the calculated motion analogous degree, to calculate an exercise quantity of the user's motion, and a display apparatus thereof.

According to an aspect of an exemplary embodiment, a method for measuring exercise quantity includes photographing a user's motion to obtain a motion image; calculating a motion analogous degree representing a comparison between the motion image and a sample motion image, wherein the sample motion image is previously stored in association with a sample value of exercise quantity; and calculating an exercise quantity of the user's motion based on the motion analogous degree and the sample value of exercise quantity.

The method may further include photographing the sample motion to obtain the sample motion image; using the sample motion image to calculate coordinates of a plurality of body points of the user, thus modeling the sample motion; obtaining the sample value of exercise quantity based on the modeled sample motion; and storing the sample motion image in association with the sample value of exercise quantity.

The obtaining the sample value of exercise quantity may include defining the sample value of exercise quantity in units of Metabolic equivalent of task (METs).

The obtaining the sample value of exercise quantity may include measuring a pulse of a user, and the storing the sample motion image may include storing information regarding the sample motion, image in association with the sample value of exercise quantity, and contents where the sample motion is executed. Herein, the contents is exercise contents which may map and store the sample motion image regarding the user's particular sample and the exercise quantity of the particular sample.

The calculating the motion analogous degree may include dividing the motion image into a plurality of blocks; and comparing movements of the plurality of blocks of the motion image with movements of a plurality of blocks of the sample motion image.

The plurality of blocks may include a left leg block, a right leg block, a left arm block, a right arm block and a main body block.

The calculating the exercise quantity of the user's motion may include calculating a first exercise quantity of the user's motion based on the motion analogous degree and the sample value of exercise quantity; and calculating a final exercise quantity of the user's motion based on at least one of the first exercise quantity, an exercise time, a weight of the user, gender information of the user and an age of the user.

The calculating the final exercise quantity may include using a formula:

$$\text{final exercise quantity (cal)} = \frac{1.20(METs-1)*T*W*A}{Wm}$$

wherein METs is the first exercise quantity value, T is the exercise time in units of minutes, W is the weight of the user in units of kilograms, A is an age information coefficient, and Wm is a gender information coefficient.

The obtaining the motion image may use a 3D camera.

According to an aspect of another exemplary embodiment, a display apparatus may include an image obtaining unit which photographs a user's motion to obtain a motion image; a motion analogous degree calculating unit which calculates a motion analogous degree representing a comparison between the motion image and a sample motion image, wherein the sample motion image is previously stored in association with a sample value of exercise quantity; and an exercise quantity calculating unit which calculates an exercise quantity of the user's motion based on the motion analogous degree and the sample value of exercise quantity.

The apparatus may further include an image obtaining unit which photographs the sample motion to obtain a sample motion image; a modeling unit which uses the sample motion image to calculate coordinates of a plurality of body points of the user to model the sample motion; an exercise quantity obtaining unit which obtains the sample value of exercise quantity based on the modeled sample motion; and a storage unit which stores the sample motion image in association with the sample value of exercise quantity.

The exercise quantity obtaining unit may obtain the sample value of exercise quantity in units of Metabolic equivalent of task (METs).

The exercise quantity obtaining unit may measure the user's pulse to obtain exercise quantity of the sample motion, and the storage unit may store information regarding the sample motion image in association with the sample value of exercise quantity, and contents where the sample motion is executed.

The motion analogous degree calculating unit may divide the motion image into a plurality of blocks; compares movements of the plurality of blocks of the motion image with movements of a plurality of blocks of the sample motion image.

The plurality of blocks may consist of a left leg block, a right leg block, a left arm block, a right arm block and a main body block.

The exercise quantity calculating unit may calculate a first exercise quantity of the user's motion based on the motion analogous degree and the sample motion image, and calculate a final exercise quantity based on at least one of the first exercise quantity, an exercise time, a weight of the user, gender information of the user and an age of the user.

The exercise quantity calculating unit may calculate the final exercise quantity using a formula:

$$\text{final exercise quantity (cal)} = \frac{1.20(METs - 1) * T * W * A}{Wm}$$

wherein METs is the first exercise quantity value, T is the exercise time in units of minutes, W is the weight of the user in units of kilograms, A is an age information coefficient, and Wm is a gender information coefficient.

The motion image obtaining unit may include a 3D camera.

The above and/or other aspects and advantages will be more apparent by describing certain exemplary embodiments with reference to the accompanying drawings, in which:

FIG. 1 is a view illustrating a display apparatus for measuring quantity of exercise, according to an exemplary embodiment of the present invention;

FIG. 2 is a block diagram illustrating a configuration of a display apparatus for measuring quantity of exercise, according to an exemplary embodiment of the present invention;

FIG. 3 is a flowchart for explaining a method for measuring a quantity of exercise, according to an exemplary embodiment of the present invention;

FIGs. 4 and 5 are flowcharts for explaining a method for mapping and storing a sample motion image and quantity of exercise, according to an exemplary embodiment of the present invention;

FIG. 6 is a health management system which includes a display apparatus and server for health management, according to an exemplary embodiment of the present invention;

FIG. 7 is a block diagram illustrating a configuration of a display apparatus for health management, according to an exemplary embodiment of the present invention;

FIGs. 8A to 12 are views illustrating a UI provided for managing the health of a user in a display apparatus for health management, according to various exemplary embodiments of the present invention;

FIG. 13 is a block diagram illustrating a configuration of a server included in a health management system, according to an exemplary embodiment of the present invention; and

FIGs. 14 to 16 are flowcharts for illustrating a health management method, according to various exemplary embodiments of the present invention.

Certain exemplary embodiments are described in higher detail below with reference to the accompanying drawings.

In the following description, like drawing reference numerals are used for the like elements, even in different drawings. The matters defined in the description, such as detailed construction and elements, are provided to assist in a comprehensive understanding of exemplary embodiments. However, exemplary embodiments can be practiced without those specifically defined matters. Also, well-known functions or constructions are not described in detail since they would obscure the application with unnecessary detail.

Although a few embodiments are shown and described, it would be appreciated by those skilled in the art that changes may be made in these embodiments without departing from the invention, the scope of which is defined in the claims.

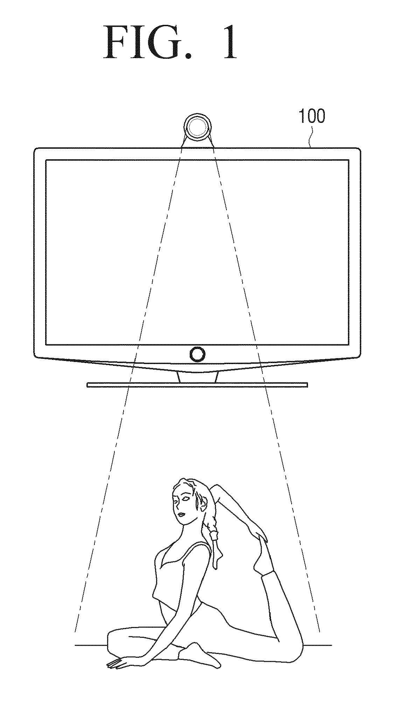

FIG. 1 is a view illustrating a display apparatus 100 for measuring a quantity of exercise, according to an exemplary embodiment of the present invention.

A display apparatus 100 according to an exemplary embodiment of the present invention maps and pre-stores a sample motion image and exercise quantity of a particular value. Herein, the method of mapping and storing the sample motion image and the exercise quantity may be conducted using exercise quantity information of motions defined in METs (Metabolic equivalent of task). Otherwise, the display apparatus 100 may execute a program for mapping and storing the sample motion and exercise quantity, and may calculate an exercise quantity value of a particular sample motion, in order to map and store the sample motion and exercise quantity.

Furthermore, as illustrated in FIG. 1, the display apparatus 100 photographs the user's overall motions using a 3D camera, and obtains a motion image.

In addition, the display apparatus 100 calculates a motion analogous degree of the obtained motion image and the pre-stored sample motion image. Herein, the display apparatus 100 may compare a coordinate value of the obtained motion image and a coordinate value of the sample motion image, to calculate the motion analogous degree.

In addition, the display apparatus 100 uses the calculated motion analogous degree to calculate exercise quantity information of the user's motion. Herein, the display apparatus 100 may calculate final exercise quantity information using not only the exercise quantity information calculated using the motion analogous degree but also using various elements such as exercise time, a user's weight, and a user's gender information and age information.

As aforementioned, since it is unnecessary to attach an additional apparatus to the user's body, a user inconvenience is reduced, and it is possible to measure the exercise quantity using overall movements of the user.

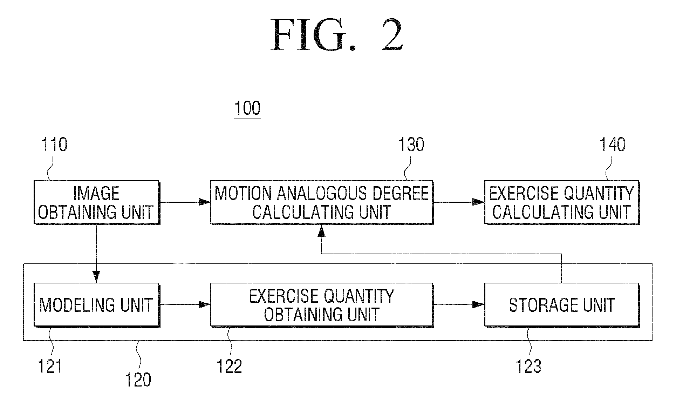

With reference to FIG. 2, the display apparatus 100 for measuring exercise quantity will be explained in more detail.

FIG. 2 is a block diagram illustrating a configuration of a display apparatus 100 according to an exemplary embodiment of the present invention. As illustrated in FIG. 2, the display apparatus 100 according to an exemplary embodiment of the present invention includes an image obtaining unit 110, a sample motion storage unit 120 which includes a modeling unit 121, an exercise obtaining unit 122, and a storage unit 123, a motion analogous degree calculating unit 130, and an exercise quantity measuring unit 140.

The image obtaining unit 110 obtains a motion image by photographing a motion of a user. Herein, the image obtaining unit 110 may obtain the motion image of the user using a 3D camera. Herein, the image obtaining unit 110 may obtain the motion image having an average of 15fPs.

In addition, the image obtaining unit 110 may obtain not only the user's motion image for measuring the exercise quantity but also a sample motion image regarding a sample motion.

The sample motion storage unit 120 maps and pre-stores the exercise quantity of the sample image and sample motion before measuring exercise quantity of a particular motion. More specifically, the sample motion storage 120 includes a modeling unit 121, exercise quantity obtaining unit 122 and storage unit 123.

The modeling unit 121 calculates coordinates of a plurality of the user's body points included in the sample motion image, and models the sample motion of the user. More specifically, the modeling unit 121 measures changes during a predetermined time (for example, 1 second) of (x, y, z) coordinates of the user's plurality of body points (for example, 14 points), and models the user's sample motion.

The exercise obtaining unit 122 obtains exercise quantity of the modeled sample motion. Herein, the exercise quantity obtaining unit 122 may obtain the exercise quantity of the sample motion using the METs (metabolic equivalent of tasks). In addition, the exercise quantity obtaining unit 122 may obtain the exercise quantity of the sample motion using pulse information of the user.

In addition, the storage unit 123 maps and stores the sample motion image and the obtained exercise quantity. Herein, the storage unit 123 may map and store not only the sample motion image and the exercise quantity but also contents where the sample motion is executed.

Hereinbelow is a detailed explanation of the method by the sample motion storage unit 120 mapping and pre-storing the sample motion image and the exercise quantity of the sample motion.

As an exemplary embodiment of the present invention, the sample motion storage unit 120 may use the METs to map and store the sample motion image and the exercise quantity. Herein, the METs (1 METs: 3.5ml/kg/min) is a unit representing intensity of exercise, that is, quantity of oxygen necessary for maintaining a stable state. Especially, regarding a general motion, the METs value is pre-defined (for example, walking at 3km/h pace is 2METs, while jogging at 9km/h pace is 8METs). METs values for general user motions are as in table 1 below.

Table 1

| User motion | METs value | User motion | METs value |
|---|---|---|---|
| Sleeping | 0.9 | drum | 4.0 |
| Sitting | 1.0 | badminton | 4,5 |
| Standing in line | 1.2 | baseball | 5.0 |
| Washing dishes | 2.3 | aerobics | 6.5 |
| Ironing | 2.3 | soccer | 7.0 |
| Stretching | 2.5 | jogging | 8.0 |
| Walking | 3.0 | cycling(fast) | 10.0 |
| Cleaning with vacuum cleaner | 3.5 | running | 15.0 |

The display apparatus 100 selects a sample motion to be modeled from among the movements defined by a user's command in the METs. In addition, when a sample motion image regarding the selected sample motion is obtained through the image obtaining unit 110, the modeling unit 121 models the user's sample motion using the sample motion image. Furthermore, the exercise quantity obtaining unit 122 obtains the exercise quantity value regarding the selected sample motion using the METs value regarding the general user motion. In addition, the storage unit 123 maps and stores the sample motion image of the selected sample motion and the obtained exercise quantity value.

As another exemplary embodiment of the present invention, the sample motion storage unit 120 may directly measure the exercise quantity regarding the user's sample motion, and map and store the sample motion image and the exercise quantity.

More specifically, the sample motion storage unit 120 executes particular contents for mapping and storing the user's sample motion image and the exercise quantity.

When the particular contents are executed, and the sample motion image regarding the particular sample motion is obtained through the image obtaining unit 110, the modeling unit 121 models the particular sample motion using the sample motion image regarding the particular sample motion. In addition, the exercise quantity obtaining unit 122 obtains the exercise quantity value regarding the particular sample motion using the user's pulse information measured in the case the user takes the particular sample motion. In addition, the storage unit 123 maps and stores the sample motion image regarding the particular motion and the obtained exercise quantity value. Herein, the storage unit 123 may also store the particular contents information executed for measuring the exercise quantity value of the sample motion and not only the sample motion image and the obtained exercise quantity value.

The motion analogous degree calculating unit 130 calculates a degree of analogy (also referred to as a "motion analogous degree") of the motion image obtained through the image obtaining unit 110 and the sample motion image pre-stored in the sample motion storage unit 120. Herein, the motion analogous degree calculating unit 130 may divide the obtained motion image into a plurality of blocks and calculate the motion analogous degree by comparing coordinate value movement regarding a plurality of blocks of the obtained motion image with coordinate value movement regarding a plurality of blocks of the pre-stored sample motion image. Herein, regarding the user's body, the plurality of blocks may consist of five blocks: left leg block, right leg block, left arm block, right arm block, and main body block. However, this is merely an exemplary embodiment, and thus the user's body may be divided into a plurality of blocks other than the five blocks.

In particular, in a case where the pre-stored sample motion image is plural, the motion analogous degree calculating unit 130 may calculate both the analogous degree of the motion image and the plurality of sample motion images.

The exercise quantity calculating unit 140 may calculate the exercise quantity regarding the user motion using the motion analogous degree calculated in the motion analogous degree calculating unit 140.

In particular, in a case where the user motion is a motion defined in the METs, the exercise quantity calculating unit 140 may calculate a first exercise quantity value regarding the user motion using the motion analogous degree. Herein, the first exercise quantity value is the METs value, which may be calculated by the mathematical formula 1 below.

Mathematical formula 1:

$$(M1 * S1) + (M2 * S2) + (M3 * S3) + ... + (Mn * Sn) = METs$$

Herein, Mn is the METs value of the $n^{th}$ sample motion, and Sn is the analogous degree of the motion image and the $n^{th}$ sample motion image.

In addition, the exercise quantity calculating unit 140 may calculate not only the first exercise quantity value but also a final exercise quantity using at least one of exercise time, user's weight, user's gender information and age information. Herein, the final exercise quantity value is calorie, and may be calculated by the mathematical formula 2 below.

## Mathematical formula 2:

$$\text{Final exercise quantity (cal)} = \frac{1.20(METs - 1) * T * W * A}{Wm}$$

Herein, METs may be the first exercise quantity value, T may be the exercise time (min), W may be the weight (kg), A may be the age information coefficient, and Wm may be the gender information coefficient (male: 63, female: 52).

The age information coefficient may be defined as in table 2 below.

Table 2

| Age | Male | Female |
|-----|------|--------|
| 20- | 1.0 | 1.0 |
| 30- | 0.95 | 0.95 |
| 40~ | 0.93 | 0.91 |
| 50~ | 0.95 | 0.90 |
| 60~ | 0.91 | 0.90 |
| 70~ | 0.89 | 0.89 |

However, in a case where the user's motion is a motion that has been mapped with the exercise quantity calculated by the particular contents and then stored, and not by the motion defined in the METs, the exercise quantity calculating unit 140 may directly calculate the final exercise quantity value regarding the user motion using the motion analogous degree. Herein, the final exercise quantity value is a calorie unit.

Especially, the exercise quantity calculating unit 140 may calculate the final exercise quantity value regarding the user motion using the mathematical formula 3 below.

## Mathematical formula 3:

$$(C1 * S1) + (C2 * S2) + (C3 * S3) + ... + (Cn * Sn) = \text{calorie}$$

Herein, Cn is a calorie value of the $n^{th}$ sample motion, Sn is the analogous degree of the motion image and the $n^{th}$ sample motion image.

As aforementioned, the display apparatus 100 doesn't need an additional apparatus attached, and thus user inconvenience is reduced, and it is possible to measure the exercise quantity using the overall movements of the user.

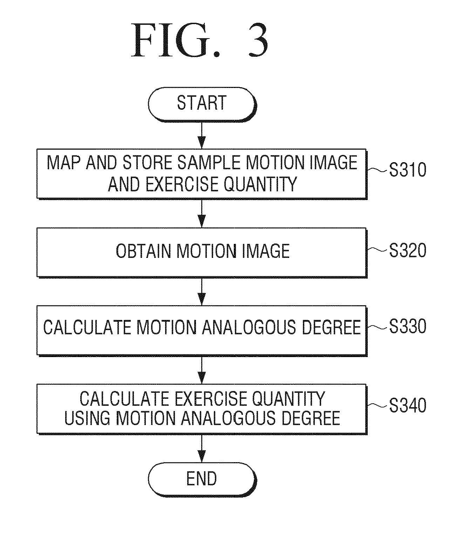

Hereinbelow is a detailed explanation on a method for measuring exercise quantity, with reference to FIGs. 3 to 5.

FIG. 3 is a flowchart for explaining a method for measuring exercise quantity, according to an exemplary embodiment of the present invention.

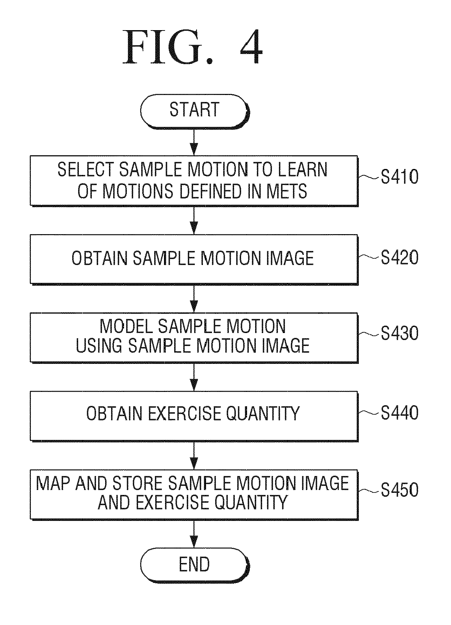

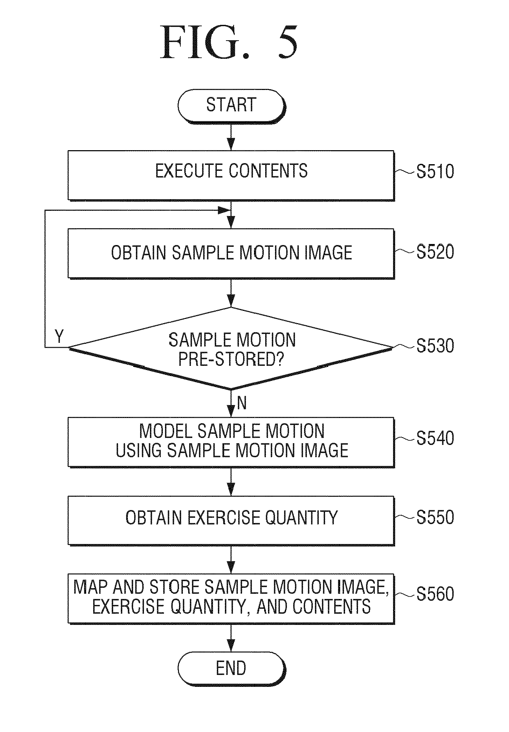

First of all, the display apparatus 100 maps and stores the sample motion image and exercise quantity (S310). Herein, the method of mapping and storing the sample motion image and exercise quantity will be explained with reference to FIGs. 4 and 5. FIG. 4 is a flowchart for explaining the method for mapping and storing the sample motion image and exercise quantity using the METs, according to an exemplary embodiment of the present invention.

First of all, the display apparatus 100 selects the sample motion to be learned of among the motions defined in METs according to the user's input (S410). For example, the display apparatus 100 may select the stretching motion which is one of the motions defined in the METs as the sample motion, according to the user's input.

In addition, the display apparatus 100 obtains the sample motion image corresponding to the selected sample motion (S420). Herein, the display apparatus 100 may obtain the sample motion image using the 3D camera.

In addition, the display apparatus 100 models the sample motion using the sample motion image (S430). More specifically, the display apparatus 100 may measure changes during the pre-set time (for example 1 second) regarding (x, y, z) coordinates of the plurality of body points (for example, 14 points) of the user, and model the user's sample motion.

In addition, the display apparatus 100 obtains the exercise quantity using the METs (S440). More specifically, the display apparatus 100 may obtain the exercise quantity corresponding to the motion pre-defined in the METs as the exercise quantity value of the selected sample motion.

In addition, the display apparatus 100 maps and stores the sample motion image and exercise quantity (S450). Herein, the stored exercise quantity may be in METs units.

FIG. 5 is a flowchart for explaining a method for mapping and storing the sample motion image and exercise quantity using contents, according to another exemplary embodiment of the present invention.

First of all, the display apparatus 100 executes the contents according to the user's command (S510). Herein, the contents is exercise contents which may map and store the sample motion image regarding the user's particular sample and the exercise quantity of the particular sample.

In addition, the display apparatus 100 obtains the sample motion image corresponding to the selected sample motion (S520). Herein, the display apparatus 100 may obtain the sample motion image using the 3D camera.

In addition, the display apparatus 100 determines whether or not the obtained sample motion is pre-stored (S530). More specifically, the display apparatus 100 may determine whether or not the obtained sample motion is pre-stored by determining whether or not a sample motion image which is the same or similar to the obtained sample motion image is pre-stored.

If the obtained sample motion is a pre-stored image (SS30-Y), the display apparatus 100 obtains the sample motion image again (S520).

If the obtained sample motion image is not a pre-stored image (S530-N), the display apparatus 100 models the sample motion using the sample motion image (S540). More specifically, the display apparatus 100 may model the user's sample motion by measuring changes during the pre-set time (for example, 1 second) regarding coordinates (x, y, z) of the plurality of body points (for example, 14 points).

In addition, the display apparatus 100 obtains the exercise quantity using the user's pulse information (S550). More specifically, the display apparatus 100 may calculate the user's pulse information using the pulse measuring apparatus, and obtain it as the exercise quantity of the particular sample motion using the calculated pulse information.

In addition, the display apparatus 100 maps and stores the sample motion image and the exercise quantity (S560). Herein, the display apparatus 100 may not only store the sample motion image and the exercise quantity but also the contents information as well. Herein, the stored exercise quantity may in calorie unit.

As illustrated in FIGs. 4 and 5, the display apparatus 100 may map and store the sample motion image and exercise quantity.

With reference to FIG. 3, the display apparatus 100 obtains the motion image (S320). Herein, the display apparatus 100 may obtain the motion image using the 3D camera. In addition, the display apparatus 100 calculates the motion analogous degree of the motion image and the sample motion image (S330). More specifically, the display apparatus 100 may calculate the motion analogous degree by comparing coordinate value movement regarding a plurality of blocks of the obtained motion image with coordinate value movement regarding a plurality of blocks of the pre-stored sample motion image. Herein, regarding the user's body, the plurality of blocks may consist of five blocks: left leg block, right leg block, left arm block, right arm block, and main body block. However, this is merely an exemplary embodiment, and thus the user's body may be divided into a plurality of blocks other than the five blocks.

Especially, in a case where the pre-stored sample motion image is plural, the display apparatus 100 may calculate both the analogous degree of the motion image and the plurality of sample motion images.

In addition, the display apparatus 100 calculates the exercise quantity using the calculated analogous degree (S340). Herein, if the motion corresponding to the motion image is a motion defined in the METs, the display apparatus 100 calculates the first exercise quantity having the METs unit using the motion analogous degree with the sample motion image. In addition, the display apparatus 100 may not only calculate the first exercise quantity but also calculate the final exercise quantity of calorie unit using at least one of the exercise time, user's weight, user's gender information and age information. However, in the case where the motion corresponding to the motion image is a motion defined by the contents, the display apparatus 100 may calculate the final exercise quantity of calorie unit directly using the motion analogous degree with the sample motion image.

Due to the aforementioned method for measuring the exercising quantity, the display apparatus 100 doesn't need an additional apparatus attached, and thus user inconvenience is reduced, and it is possible to measure the exercise quantity using the overall movements of the user.

Hereinbelow is an explanation on various exemplary embodiments providing health management services where the method for measuring the exercise quantity of the present invention is applied.

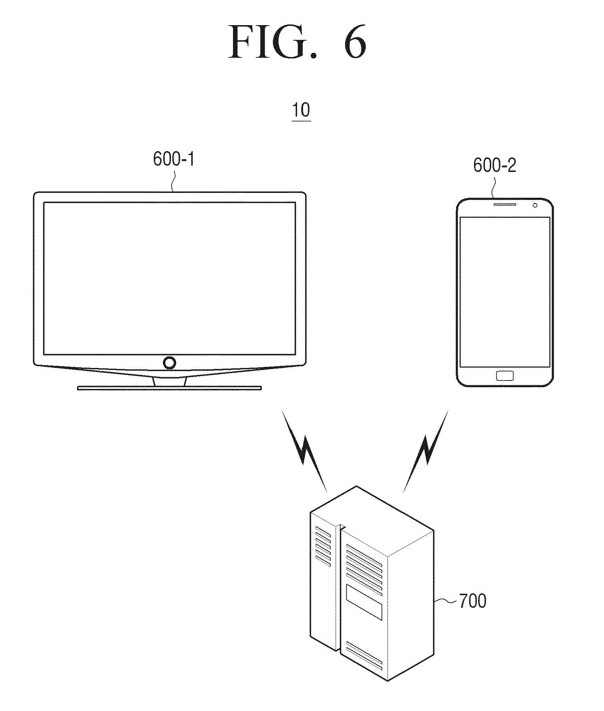

With reference to FIG. 6, the health management system 10 includes a plurality of display apparatuses 600-1, 600-2 and a health management server 700. Meanwhile, in FIG. 6, the display apparatus may be a TV 600-1 or a mobile phone 600-2, but these are merely exemplary, and thus the display apparatus may be embodied as a PC, s notebook PC, s tablet PC, a PDA, or the like.

The display apparatus 600 performs a user certification. Herein, the user certification may use at least one of ID/password, face recognition, iris recognition and fingerprint recognition. Through user certification, the user may be provided with the health management service according to an exemplary embodiment of the present invention using the plurality of display apparatuses 600-1, 600-2, regardless of time and place.

In addition, the display apparatus 600 obtains a user's body information and a user's exercise goal information. Herein, the user's body information may include the user's height and weight, and the display apparatus 600 may obtain the user's body information using a digital scale and a depth camera. Herein, the user's body information including the user's height and weight is merely exemplary, and thus, the user's body information may further include information such as the user's pulse, blood pressure, body temperature, and brain waves.

In addition, the display apparatus 600 obtains the user's exercise goal information based on to the user's setting. Herein, the exercise goal information may include at least one of the user's weight goal (or weight loss goal), burning calories, exercise period, and exercise contents used by the user.

Herein, the user may obtain the user's body information and exercise goal information via only the display apparatus 600, but this is merely exemplary, and thus it is possible to obtain the user's body information and exercise goal information through the plurality of display apparatuses 600-1, 600-2.

In addition, the display apparatus 600 transmits the obtained user's body information and exercise goal information to the health management server 700.

The health management server 700 receives and stores the user's body information and the exercise goal information transmitted from the display apparatus 600.

In addition, the display apparatus 600 measures the exercise quantity information in the method explained in FIGs. 1 to 5. Especially, the display apparatus 600 may measure the exercise quantity information using the contents, program, and application which may measure the user's exercise quantity information. In addition, the display apparatus 600 may measure the exercise quantity information using a depth camera, an arm band, a pulsimeter, or a blood pressure meter. Herein, the exercise quantity information may include at least one of the exercise contents information used by the user, a user's exercise time information, and burning calorie information.

The display apparatus 600 may transmit the measured exercise quantity information to the health management server 700.

The health management server 700 stores the exercise quantity information received through the plurality of display apparatuses 600.

The health management server 700 generates health management data for generating a health management user interface (UI) using the received user's body information, exercise goal information, and exercise quantity information.

In addition, when a request for transmission of the health management data is received from the display apparatus 600, the health management server 700 transmits the health management data to the display apparatus 600. The health management data may include the user's changed body information, whether or not the user's goal has been reached, and recommended exercise contents.

In addition, the display apparatus 600 generates and displays the health management UI using the received health management data. Herein, the health management UI may use at least one exercise contents and may display the measured exercise quantity information by date or contents. In addition, the health management UI may display the user's changed body information and information regarding whether or not the user's exercise goal has been reached.

By the aforementioned health management system 10, the user becomes able to intuitively check his/her change of health state, and manage his/her health comprehensively using the plurality of exercise contents.

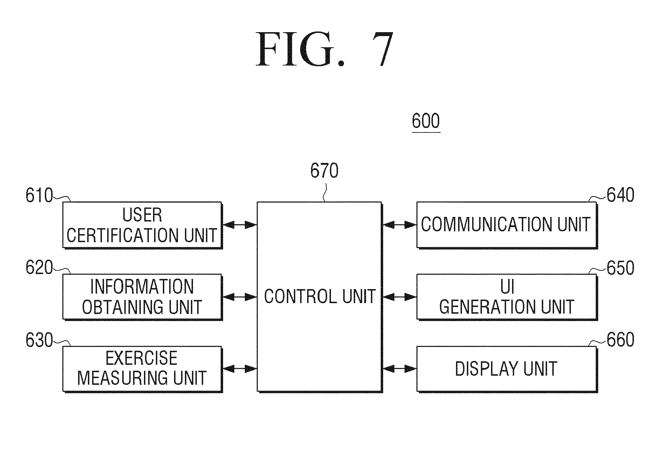

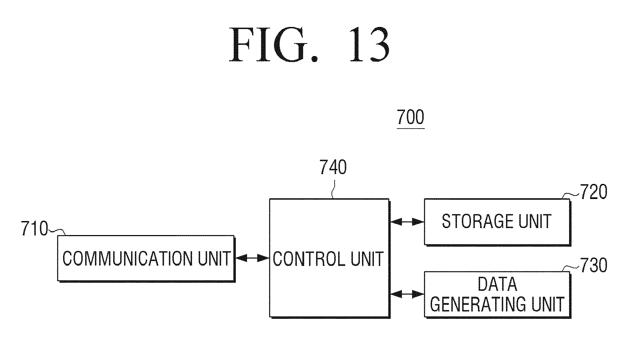

Hereinbelow is an explanation of the display apparatus 600 and the health management server 700 according to an exemplary embodiment of the present invention, with reference to FIGs. 7 and 13.

FIG. 7 is a block diagram illustrating a configuration of the display apparatus 600 according to an exemplary embodiment of the present invention. As illustrated in FIG. 7, the display apparatus 600 includes a user certification unit 610, an information obtaining unit 620, an exercise quantity measuring unit 630, a communication unit 640, a UI generating unit 650, a display unit 660, and a control unit 670.

The user certification unit 610 certifies the user who is to receive the health management service. Herein, the user certification unit 610 may certify the user using an ID/password, but this is merely exemplary, and thus the user may certify the user using face recognition, iris recognition, and/or fingerprint recognition etc.

The information obtaining unit 620 obtains the user's body information and exercise goal information. More specifically, the information obtaining unit 620 may obtain the user's body information using one or more auxiliary devices such as a digital scale and a depth camera. Especially, the information obtaining unit 620 may obtain the user's weight information using a digital scale, and obtain the user's height information using a depth camera. However, this is merely exemplary, and the information obtaining unit 620 may obtain the user's pulse information using a pressuremeter, and may obtain the user's pulse information using a pulsimeter.

In addition, the information obtaining unit 620 may obtain the user's exercise goal information through the user's setting. Herein, the user's exercise goal information may include at least one of the user's weight goal (or weight loss goal), burning calories, an exercising period, and exercise contents used by the user.

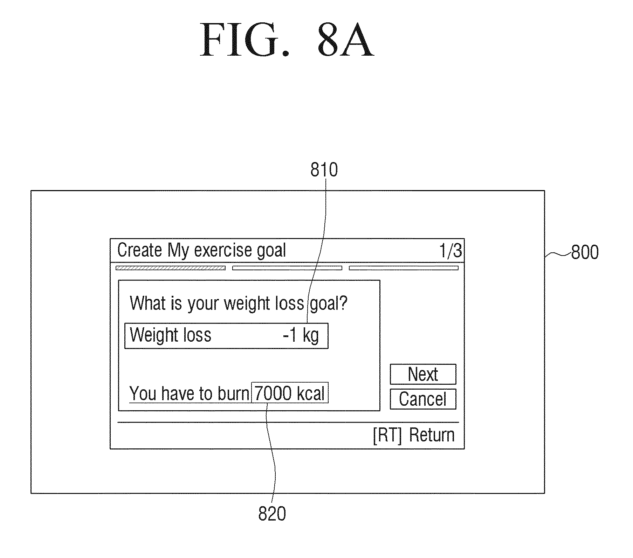

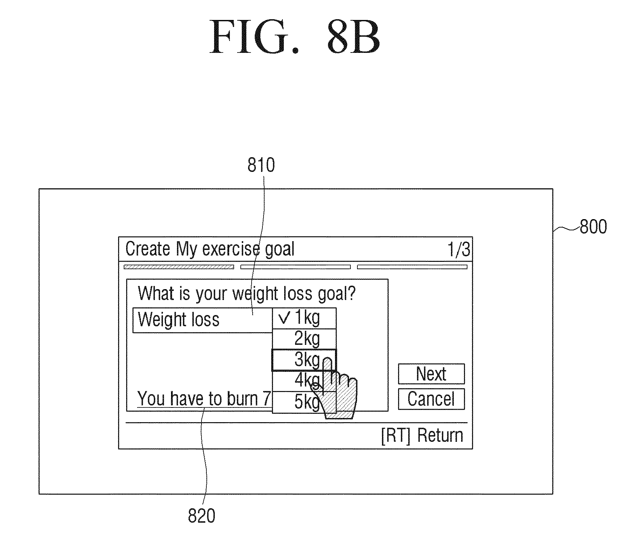

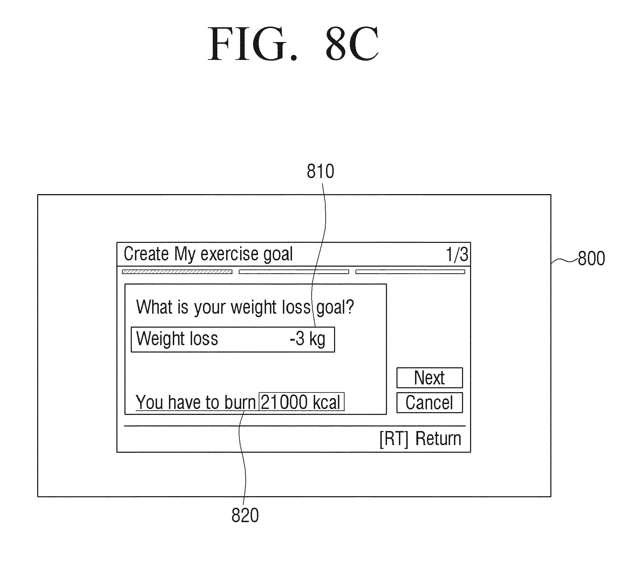

For example, the information obtaining unit 620 may input the user's weight loss goal using the exercise goal input UI, illustrated in FIGs. 8a to 8b. In a case where the weight loss goal is 1kg as illustrated in FIG. 8a, the exercise goal input UI 800 may display the weight loss goal and a burning calorie quantity corresponding to the weight loss goal through a plurality of menus 810, 820. Herein, when a menu 810 displaying the weight loss goal is selected, as illustrated in FIG 8b, the user may change the weight loss goal that he/she intends to lose. In addition, in a case where the user intends to lose 3kg, the user may select 3kg through the menu 810, as illustrated in FIG. 8b. Furthermore, when 3kg is selected, the exercise goal input UI 810 may display the changed weight loss goal and burning calorie quantity on the plurality of menus 810, 820.

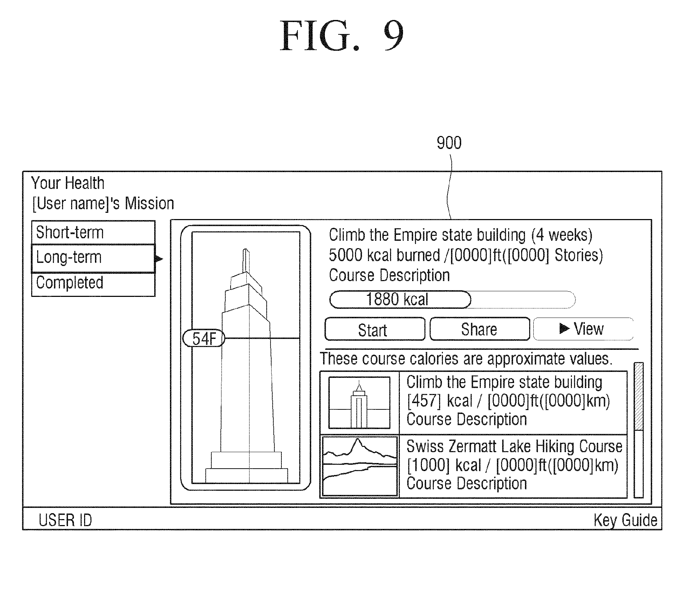

Furthermore, the information obtaining unit 610 becomes able to set the exercise goal using a mission list 900 illustrated in FIG. 9. Herein, the mission list 900 is a list which displays a mission corresponding to the calorie quantity that the user intends to burn, thereby guiding the burning calorie quantity to the user. For example, as illustrated in FIG. 9, the mission list 900 may guide that 457 kcal of calorie has to be burned to climb the Empire State Building. Through such a mission list, the user becomes able to know his/her burning calorie quantity easily.

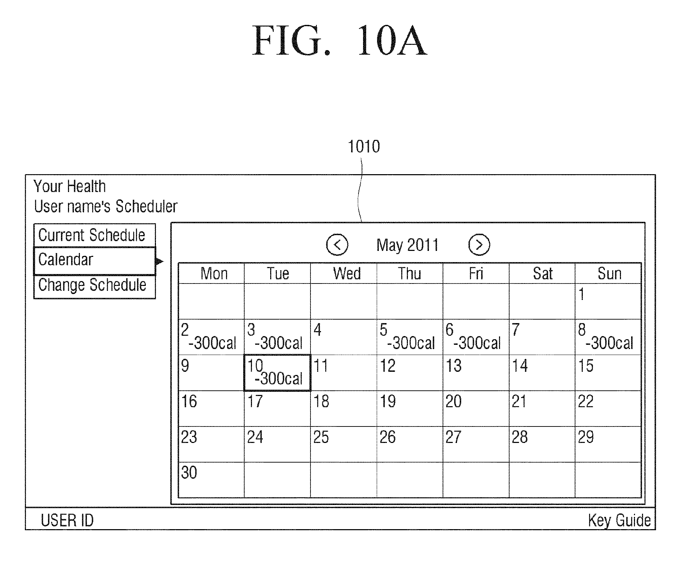

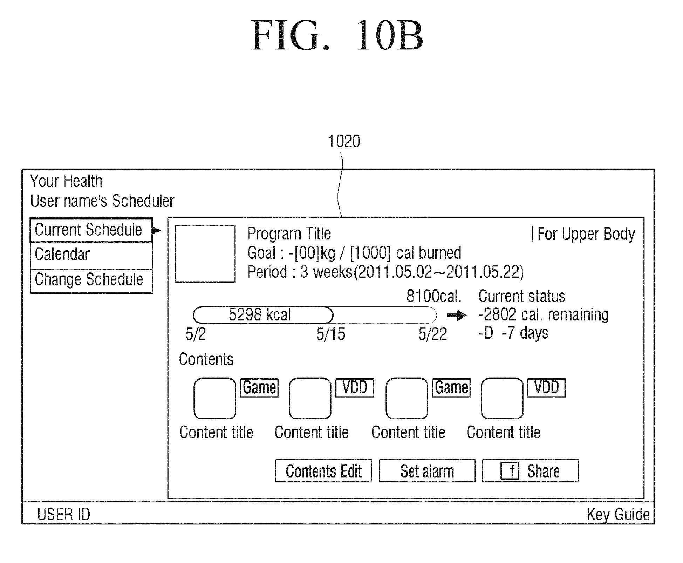

In addition, the information obtaining unit 610 may set the exercise goal quantity and the exercise contents for the user to use by date. For example, the information obtaining unit 610 may set the exercise goal quantity by date using a calendar UI 1010 as illustrated in FIG. 10a, and may set an exercise program, application, and contents that the user will use using an exercise contents setting UI 1020 as illustrated in FIG. 10b.

The exercise quantity measuring unit 630 measures the user's exercise quantity information. Herein, the user's exercise quantity information includes at least one of the exercise contents information that the user used, the user' exercise time information, and the burned calorie information etc. Especially, the exercise quantity measuring unit 630 may measure the exercise quantity information regarding the user's motion using the method for measuring exercise quantity as illustrated in FIGs. 1 to 5. That is, the exercise quantity measuring unit 630 may include the image obtaining unit 110, the sample motion storage unit 120 which includes the modeling unit 121, exercise quantity obtaining unit 122 and storage unit 120, the motion analogous degree calculating unit 130, and the exercise quantity measuring unit 140.

The communication unit 640 performs communication with an external health management server 700. Herein, the communication unit 740 may use a Wi-fi module and Ethernet etc., but it is not limited thereto.

Especially, the communication unit 640 may transmit the user's body information, user's exercise goal information, and user's exercise quantity information output from the information obtaining unit 620 and the exercise quantity measuring unit 630. In addition, the communication unit 640 may receive the health management data from the health management server 700. Herein, the health management data may include the user's changed body information, whether or not the user reached the goal, and recommended exercise contents. However, the aforementioned health management data is merely exemplary, and thus the health management data may be data including the user's body information, the user's exercise goal information, and the user's exercise quantity information, and not processed data.

The UI generating unit 650 generates various UIs for receiving the health management service. Especially, the UI generating unit 650 may generate various UIs for inputting the exercise goal as illustrated in FIG. 10b.

In addition, the UI generating unit 650 may generate the health management UI using the health management data received through the communication unit 640. The health management UI may display the exercise quantity information measured using at least one contents by date or contents using graphs, and also the user's changed body information and whether or not the user's exercise goal has been reached.

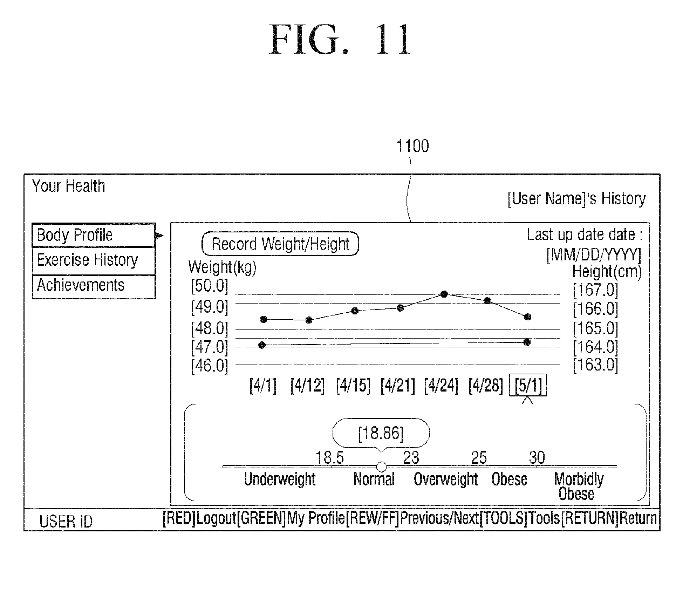

For example, the health management UI 1100 may display the user's body change information through a graph by date as illustrated in FIG. 11. In addition, the health management UI 1200 may display the calorie quantity burned by the user by date, and also the contents that the user used and the calorie quantity that the user burned using the corresponding contents.

The display unit 660 displays an image data signal processed by a control of the control unit 670. In addition, the display unit 670 may display various UIs generated in the UI generating unit 650.

The control unit 670 controls the overall operations of the display apparatus 600 according to the input user's command. In addition, the control unit 670 controls the user certification unit 610, information obtaining unit 620, exercise quantity information measuring unit 630, communication unit 640, UI generating unit 650, and display unit 660.

Besides, the display apparatus 600 may further include a contents recommending unit (not illustrated) which analyzes body changes according to the user's exercise contents and each of the exercise contents, and provides the recommended exercise contents to the user.

As aforementioned, it is possible to intuitively check one's change of the health state and comprehensively manage one's health using the plurality of exercise contents through the display apparatus 600 according to one or more exemplary embodiments of the present invention.

FIG. 13 is a block diagram illustrating a configuration of the health management server 700, according to an exemplary embodiment of the present invention. As illustrated in FIG. 13, the health management server 700 includes a communication unit 710, storage unit 720, data generating unit 730, and control unit 740.

The communication unit 710 performs communication with a plurality of display apparatuses 600-1, 600-2, .... Especially, it is possible to receive the user's body information, user's exercise goal information, and user's exercise quantity information from the plurality of display apparatuses 600-1, 600-2,.. of the communication unit 710. In addition, the communication unit 710 may transmit the health management data generated in the data generating unit 630 to the display apparatus 600.

The storage unit 720 stores the user's body information, user's exercise goal information, and user's exercise quantity information received from the plurality of display apparatuses 600-1, 600-2, .. Herein, the storage unit 720 may store the plurality of aforementioned information per user.

The data generating unit 730 may generate the health management data using the user's body information, user's exercise goal information, and user's exercise quantity information stored in the storage unit 720. Herein, the health management data may include the user's changed body information, whether or not the user reached the goal, and recommended exercise contents etc. However, the aforementioned health management data is merely an exemplary embodiment, and thus the health management data may be data including the user's body information, user's exercise goal information, and user's exercise quantity information and not processed data. The control unit 740 controls the overall functions and operations of the health management server 700 according to a control by a health management server provider. Especially, the control unit 740 may control the communication unit 710, storage unit 720 and data generating unit 730 for managing the user's health.

As aforementioned, the user becomes able to manage one's health using the plurality of display apparatuses 600-1, 600-2, ... regardless of time and place.

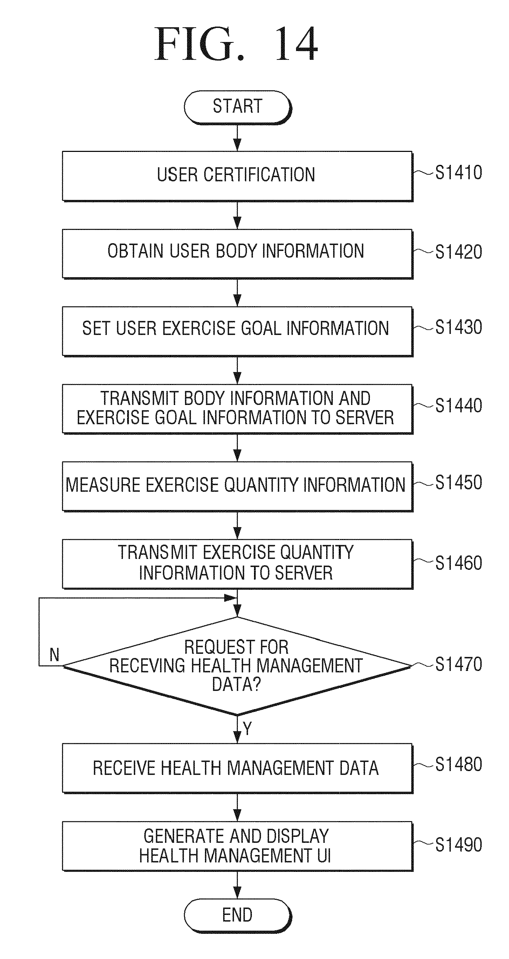

Hereinbelow is an explanation of the health management method according to various exemplary embodiments of the present invention with reference to FIGs. 14 to 16.

FIG. 14 is a flowchart for explaining the health management method of the display apparatus 600 according to an exemplary embodiment of the present invention.

First of all, the display apparatus 600 certifies the user (S1410). Herein, the display apparatus 600 may certify the user using the ID/password, face recognition, iris recognition, and fingerprint recognition.

In addition, the display apparatus 600 obtains the user's body information (S1420). Herein, the user body information may include at least one of the user's height and weight, and the display apparatus 600 may obtain the user's height information using the depth camera, and may obtain the user's weight information using the digital scale.

In addition, the display apparatus 600 sets the user's exercise goal information (S1430). Herein, the user exercise goal information may include at least one of the user's weight goal, burning calorie quantity, exercise period, and the exercise contents used by the user. Especially, the display apparatus 600 may input the exercise goal information through various exercise goal input UIs as illustrated in FIGs. 8a to 10b.

In addition, the display apparatus 600 transmits the obtained body information and exercise goal information to the server 700 (S1440).

In addition, the display apparatus 600 measures the exercise quantity information (S1450). Herein, as explained in FIGs. 1 to 5, the display apparatus 600 may measure the exercise quantity information using the method for measuring the exercise quantity as illustrated in FIGs. 1 to 5. Herein, the exercise quantity information may include not only the burning calorie quantity, but also additional information such as the contents information and exercise time information.

In addition, the display apparatus 600 transmits the calculated exercise quantity information to the server 700 (S1460).

In addition, the display apparatus 600 determines whether or not a request for receiving health management data has been made from the user (S1470).

When the request for receiving the health management is input (S1470-Y), the display apparatus 600 receives the health management data from the server 700 (S1480). Herein, the health management data may include the user's changed body information, whether or not the user reached the goal, and recommended exercise contents. However, such aforementioned health management data is merely exemplary, and thus the health management data may be data including the user's body information, user's exercise goal information, and user's exercise quantity information and not processed data.

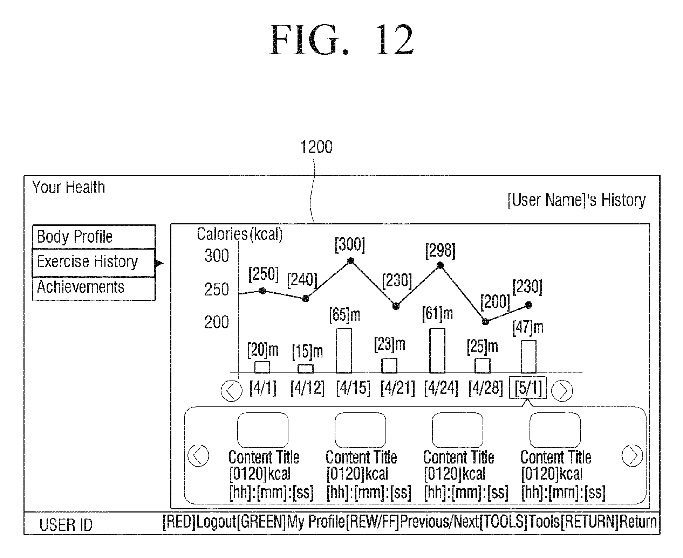

Furthermore, the display apparatus 600 generates and displays the health management UI (S1490). Herein, the health management UI may display the exercise quantity information measured using at least one contents by date or contents using graphs, and also the user's changed body information and whether or not the user's exercise goal has been reached. For example, the health management UI may be UI such as that illustrated in FIGs. 11 and 12.

According to such a display apparatus 600, the user becomes able to receive the health management service comprehensively using the plurality of exercise contents. In addition, the user becomes able to intuitively check his/her health state.

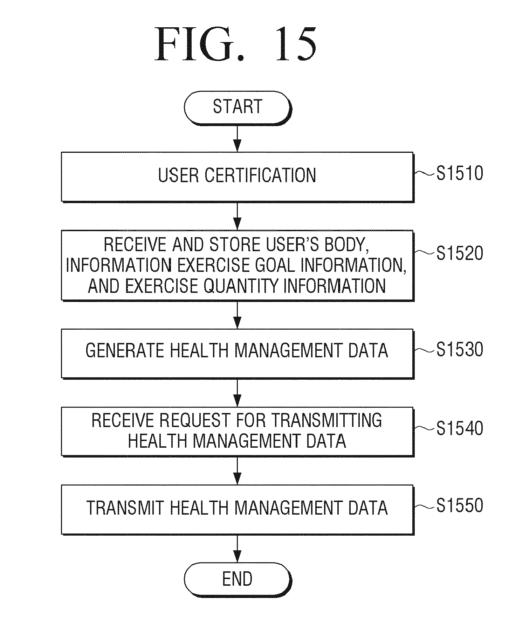

FIG. 15 is a flowchart for explaining the health management method of the health management server 700, according to an exemplary embodiment of the present invention.

First of all, the health management server 700 certifies the user (S1510). Herein, the user is a user certified from the display apparatus, who receives the health management service.

In addition, the health management server 700 receives the user's body information, exercise goal information, and exercise quantity information from the display apparatus 600, and stores them (S1520). Herein, the user's body information, exercise goal information and exercise quantity information may be received from the plurality of display apparatuses 600-1, 600-2, ....

In addition, the health management server 700 generates the health management data (S1530). Herein, the health management data may include the user's changed body information, whether or not the user reached the goal, and recommended exercise contents, but may also be data including the user's body information, user's exercise goal information, and user's exercise quantity information, and not processed data.

In addition, the health management server 700 receives the request for transmitting health management data from the display apparatus 600 (S1540).

When the request for transmitting health management data is received, the health management server 700 transmits the health management data to the external display apparatus 600 (S1550).

Through the aforementioned health management server 700, the user becomes able to manage one's health using the plurality of display apparatuses 600-1,600-2 regardless of place and time.

Meanwhile, in the aforementioned exemplary embodiments, it has been explained that the health management data is generated before the health management data transmission request, but these are merely exemplary, and thus it is possible to generate the health management data when the health management data transmission request is received.

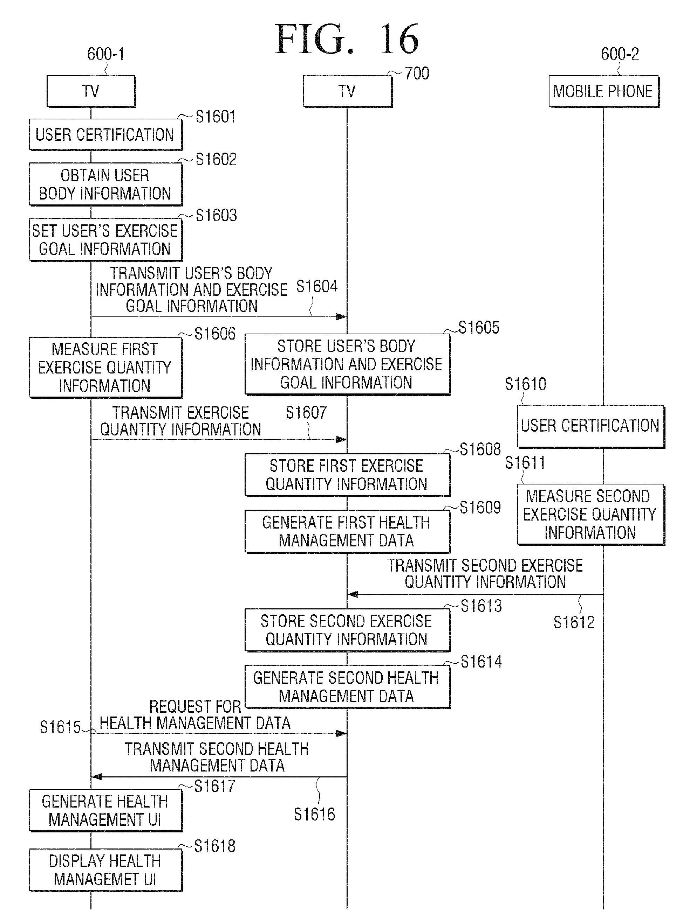

FIG. 16 is a flowchart for explaining the health management method of the health management system 10, according to an exemplary embodiment of the present invention.

First of all, the TV 600-1 performs the user certification (S1601). In addition, the TV 600-1 obtains the user's body information (S1602), and sets the user's exercise goal information (S1603).

In addition, the TV 600-1 transmits the obtained user's body information and exercise goal information to the server 700 (S1604).

The server 700 stores the received user's body information and exercise goal information (S1605).

In addition, the TV 600-1 measures the first exercise quantity information in the method illustrated in FIGs. 1 to 5 (S1606), and transmits the measured first exercise quantity information to the external server 700 (S1607).

The server 700 stores the received first exercise quantity information (S1608). In addition, the server 700 generates the first health management data using the received user's body information, exercise goal information and the first exercise quantity information (S1609).

In addition, the mobile phone 600-2 certifies the same user as the user certified in the TV 600-1 (S1610).

Furthermore, the mobile phone 600-2 measures a second exercise quantity information in the method illustrated in FIGs. 1 to 5 (S1611), and transmits the measured second exercise quantity information to the external server 700 (S1612).

When the second exercise quantity information is transmitted, the server 700 stores the second exercise quantity information (S1613). In addition, the server 700 generates a second health management data using the received user body information, exercise goal information, first exercise quantity information, and second exercise quantity information (S1614).

Then, the TV 600-1 requests for the health management data to the server (S1615).

When the health management data request signal is received, the server 700 transmits a latest second health management data to the TV 600-1 (S1616).

When the second health management data is received, the TV 600-1 generates the health management UI using the second health management data (S1617), and displays the generated health management UI (S1618).

Using the aforementioned health management system 10 and exercise measuring method, the user may intuitively check changes of his/her health state, and may comprehensively management his/her health using the plurality of display apparatuses 600-1,600-2.

In addition, a program code for performing the exercise quantity measuring method and health management method according to the aforementioned various exemplary embodiments may be stored in various types of recording media. More specifically, the aforementioned program code may be stored in various types of recording media which can be read in terminals such as a RAM(Random Access Memory), flash memory, ROM(Read Only Memory), EPROM (Erasable Programmable ROM), EEPROM(Electronically Erasable and Programmable ROM), register, hard disk, removable disk, memory card, USB memory, and CD-ROM.

Although a few embodiments of the present invention have been shown and described, it would be appreciated by those skilled in the art that changes may be made in these embodiments without departing from the invention, the scope of which is defined in the claims.

**Claims**

1. A method for measuring exercise quantity, the method comprising:

   photographing a user's motion, thus obtaining a motion image;
   calculating a degree of analogy of motion representing a comparison between the motion image and a sample motion image, wherein the sample motion image is previously stored in association with a sample value of exercise quantity; and
   calculating an exercise quantity of the user's motion based on the degree of analogy of motion and the sample value of exercise quantity.

2. The method according to claim 1, further comprising:

   photographing the sample motion, thus obtaining the sample motion image;
   using the sample motion image to calculate coordinates of a plurality of body points of the user, thus modeling the sample motion;
   obtaining the sample value of exercise quantity based on the modeled sample motion; and
   storing the sample motion image in association with the sample value of exercise quantity.

3. The method according to claim 2, wherein the obtaining the sample value of exercise quantity comprises defining the sample value of exercise quantity in units of metabolic equivalent of task (METs).

4. The method according to claim 2, wherein the obtaining the sample value of exercise quantity comprises measuring a pulse of the user, and
   the storing the sample motion image comprises storing information regarding the sample motion image in association with the sample value of exercise quantity, and contents where the sample motion is executed.

5. The method according to any one of claims 1 and 4, wherein the calculating the degree of analogy of motion comprises:

   dividing the motion image into a plurality of blocks; and
   comparing movements of the plurality of blocks of the motion image with movements of a plurality of blocks of the sample motion image.

6. The method according to claim 5, wherein the plurality of blocks comprise a left leg block, a right leg block, a left arm block, a right arm block and a main body block.

7. The method according to any one of claims 1 and 6, wherein the calculating the exercise quantity of the user's motion comprises:

   calculating a first exercise quantity of the user's motion based on the degree of analogy of motion and the sample value of exercise quantity; and
   calculating a final exercise quantity of the user's motion based on at least one of the first exercise quantity, an exercise time, a weight of the user, gender information of the user and an age of the user.

8. The method according to claim 7, wherein the calculating the final exercise quantity comprises using a formula:

$$\text{final exercise quantity (cal)} = \frac{1.20(METs - 1) * T * W * A}{Wm}$$

wherein METs is the first exercise quantity value, T is the exercise time in units of minutes, W is the weight of the user in units of kilograms, A is an age information coefficient, and Wm is a gender information coefficient.

9. The method according to any one of claims 1 and 8, wherein the photographing the user's motion comprises using a 3D camera.

10. A display apparatus comprising:

an image obtaining unit which photographs a user's motion, thus obtaining a motion image;
a motion analogous degree calculating unit which calculates a degree of analogy of motion representing a comparison between the motion image and a sample motion image, wherein the sample motion image is previously stored in association with a sample value of exercise quantity; and
an exercise quantity calculating unit which calculates an exercise quantity of the user's motion based on the degree of analogy of motion and with the sample value of exercise quantity.

11. The apparatus according to claim 10 further comprising:

a sample image obtaining unit which photographs the sample motion, thus obtaining the sample motion image;
a modeling unit which uses the sample motion image to calculate coordinates of a plurality of body points of the user, thus modeling the sample motion;
an exercise quantity obtaining unit which obtains the sample value of exercise quantity based on the modeled sample motion; and
a storage unit which stores the sample motion image in association with the sample value of exercise quantity.

12. The apparatus according to claim 11, wherein the exercise quantity obtaining unit obtains the sample value of exercise quantity in units of metabolic equivalent of task (METs).

13. The apparatus according to claim 11, wherein the exercise quantity obtaining unit measures a pulse of the user, and the storage unit stores information regarding the sample motion image in association with the sample value of exercise quantity, and contents where the sample motion is executed.

14. The apparatus according to any one of claims 10 and 13, wherein the motion analogous degree calculating unit divides the motion image into a plurality of blocks; compares movements of the plurality of blocks of the motion image with movements of a plurality of blocks of the sample motion image.

15. The apparatus according to any one of claims 10 and 14, wherein the exercise quantity calculating unit calculates the final exercise quantity using a formula:

$$\text{final exercise quantity (cal)} = \frac{1.20(METs-1)*T*W*A}{Wm}$$

wherein METs is the first exercise quantity value, T is the exercise time in units of minutes, W is the weight of the user in units of kilograms, A is an age information coefficient, and Wm is a gender information coefficient.

# FIG. 1

# FIG. 2

100

110

130

140

| IMAGE OBTAINING UNIT | → | MOTION ANALOGOUS DEGREE CALCULATING UNIT | → | EXERCISE QUANTITY CALCULATING UNIT |

| MODELING UNIT | → | EXERCISE QUANTITY OBTAINING UNIT | → | STORAGE UNIT |

121

120

122

123

# FIG. 3

START

MAP AND STORE SAMPLE MOTION IMAGE
AND EXERCISE QUANTITY — S310

OBTAIN MOTION IMAGE — S320

CALCULATE MOTION ANALOGOUS DEGREE — S330

CALCULATE EXERCISE QUANTITY
USING MOTION ANALOGOUS DEGREE — S340

END

# FIG. 4

START

SELECT SAMPLE MOTION TO LEARN OF MOTIONS DEFINED IN METS — S410

OBTAIN SAMPLE MOTION IMAGE — S420

MODEL SAMPLE MOTION USING SAMPLE MOTION IMAGE — S430

OBTAIN EXERCISE QUANTITY — S440

MAP AND STORE SAMPLE MOTION IMAGE AND EXERCISE QUANTITY — S450

END

# FIG. 5

START

EXECUTE CONTENTS — S510

OBTAIN SAMPLE MOTION IMAGE — S520

SAMPLE MOTION PRE-STORED? — S530

Y

N

MODEL SAMPLE MOTION USING SAMPLE MOTION IMAGE — S540

OBTAIN EXERCISE QUANTITY — S550

MAP AND STORE SAMPLE MOTION IMAGE, EXERCISE QUANTITY, AND CONTENTS — S560

END

# FIG. 6

# FIG. 7

600

670

610 USER CERTIFICATION UNIT

620 INFORMATION OBTAINING UNIT

630 EXERCISE MEASURING UNIT

CONTROL UNIT

640 COMMUNICATION UNIT

650 UI GENERATION UNIT

660 DISPLAY UNIT

# FIG. 8A

810

Create My exercise goal                    1/3    ~800

What is your weight loss goal?

| Weight loss | -1 kg |

Next

You have to burn 7000 kcal

Cancel

[RT] Return

820

# FIG. 8B

810

800

Create My exercise goal                                    1/3

What is your weight loss goal?

| Weight loss | ✓ 1kg |
|---|---|
| | 2kg |
| | 3kg |
| | 4kg |
| You have to burn 7 | 5kg |

Next

Cancel

[RT] Return

820

# FIG. 8C

810

Create My exercise goal                                    1/3

What is your weight loss goal?

Weight loss                    -3 kg

                                              Next
You have to burn 21000 kcal                   Cancel

                                       [RT] Return

800

820

# FIG. 9

900

Your Health
[User name]'s Mission

| Short-term |
| Long-term |
| Completed |

54F

Climb the Empire state building (4 weeks)
5000 kcal burned /[0000]ft([0000] Stories)
Course Description

1880 kcal

| Start | Share | ▶ View |

These course calories are approximate values.

Climb the Empire state building
[457] kcal / [0000]ft([0000]km)
Course Description

Swiss Zermatt Lake Hiking Course
[1000] kcal / [0000]ft([0000]km)
Course Description

USER ID                                            Key Guide

# FIG. 10A

1010

| Your Health | | | | | | | |
|---|---|---|---|---|---|---|---|
| User name's Scheduler | | | | | | | |

| Current Schedule | | | | May 2011 | | | |
|---|---|---|---|---|---|---|---|
| Calendar | Mon | Tue | Wed | Thu | Fri | Sat | Sun |
| Change Schedule | | | | | | | 1 |
| | 2 -300cal | 3 -300cal | 4 | 5 -300cal | 6 -300cal | 7 | 8 -300cal |
| | 9 | 10 -300cal | 11 | 12 | 13 | 14 | 15 |
| | 16 | 17 | 18 | 19 | 20 | 21 | 22 |
| | 23 | 24 | 25 | 26 | 27 | 28 | 29 |
| | 30 | | | | | | |

| USER ID | Key Guide |
|---|---|

# FIG. 10B

1020

Your Health
User name's Scheduler

| Current Schedule ▶ |
| Calendar |
| Change Schedule |

Program Title                                    | For Upper Body
Goal : -[00]kg / [1000] cal burned
Period : 3 weeks(2011.05.02~2011.05.22)

8100cal.    Current status
5298 kcal  ➡  -2802 cal. remaining
5/2                5/15              5/22    -D  -7 days

Contents

[Game]        [VDD]        [Game]        [VDD]

Content title  Content title  Content title  Content title

Contents Edit    Set alarm    [f] Share

USER ID                                              Key Guide

# FIG. 11

1100

Your Health                                              [User Name]'s History

| Body Profile |
|---|
| Exercise History |
| Achievements |

( Record Weight/Height )

Weight(kg)                                              Last up date date :
[50.0]                                                  [MM/DD/YYYY]
[49.0]                                                  Height(cm)
[48.0]                                                  [167.0]
[47.0]                                                  [166.0]
[46.0]                                                  [165.0]
                                                        [164.0]
                                                        [163.0]

[4/1]  [4/12]  [4/15]  [4/21]  [4/24]  [4/28]  [[5/1]]

[18.86]

18.5          23        25        30
Underweight   Normal  Overweight  Obese   Morbidly
                                          Obese

USER ID    [RED]Logout[GREEN]My Profile[REW/FF]Previous/Next[TOOLS]Tools[RETURN]Return

# FIG. 12

1200

Your Health

[User Name]'s History

| Body Profile |
| Exercise History ▶ |
| Achievements |

Calories(kcal)

300

[250] [240] [300] [230] [298] [200] [230]

250

200

[65]m [61]m [47]m

[20]m [15]m [23]m [25]m

⊙ [4/1] [4/12] [4/15] [4/21] [4/24] [4/28] [5/1] ⊙

⊙ Content Title [0120]kcal [hh]:[mm]:[ss]   Content Title [0120]kcal [hh]:[mm]:[ss]   Content Title [0120]kcal [hh]:[mm]:[ss]   Content Title [0120]kcal [hh]:[mm]:[ss] ⊙

| USER ID | [RED]Logout[GREEN]My Profile[REW/FF]Previous/Next[TOOLS]Tools[RETURN]Return |

# FIG. 13

700

740

720

710

STORAGE UNIT

COMMUNICATION UNIT ⟷ CONTROL UNIT

730

DATA GENERATING UNIT

# FIG. 14

START

USER CERTIFICATION — S1410

OBTAIN USER BODY INFORMATION — S1420

SET USER EXERCISE GOAL INFORMATION — S1430

TRANSMIT BODY INFORMATION AND EXERCISE GOAL INFORMATION TO SERVER — S1440

MEASURE EXERCISE QUANTITY INFORMATION — S1450

TRANSMIT EXERCISE QUANTITY INFORMATION TO SERVER — S1460

REQUEST FOR RECEIVING HEALTH MANAGEMENT DATA? — S1470

N

Y

RECEIVE HEALTH MANAGEMENT DATA — S1480

GENERATE AND DISPLAY HEALTH MANAGEMENT UI — S1490

END

# FIG. 15

```
                    ┌─────────┐
                    │  START  │
                    └────┬────┘
                         │
                         ▼
    ┌─────────────────────────────────────┐
    │        USER CERTIFICATION           │──── S1510
    └─────────────────┬───────────────────┘
                      │
                      ▼
    ┌─────────────────────────────────────┐
    │    RECEIVE AND STORE USER'S BODY,   │
    │ INFORMATION EXERCISE GOAL INFORMATION,│── S1520
    │   AND EXERCISE QUANTITY INFORMATION  │
    └─────────────────┬───────────────────┘
                      │
                      ▼
    ┌─────────────────────────────────────┐
    │   GENERATE HEALTH MANAGEMENT DATA   │──── S1530
    └─────────────────┬───────────────────┘
                      │
                      ▼
    ┌─────────────────────────────────────┐
    │    RECEIVE REQUEST FOR TRANSMITTING │──── S1540
    │        HEALTH MANAGEMENT DATA       │
    └─────────────────┬───────────────────┘
                      │
                      ▼
    ┌─────────────────────────────────────┐
    │   TRANSMIT HEALTH MANAGEMENT DATA   │──── S1550
    └─────────────────┬───────────────────┘
                      │
                      ▼
                 ┌─────────┐
                 │   END   │
                 └─────────┘
```

EP 2 609 858 A1

# FIG. 16

600-1                          ~700                        600-2

**TV**              **TV**                    **MOBILE PHONE**

~S1601
USER CERTIFICATION

~S1602
OBTAIN USER
BODY INFORMATION

~S1603
SET USER'S EXERCISE
GOAL INFORMATION

TRANSMIT USER'S BODY
INFORMATION AND EXERCISE  S1604
GOAL INFORMATION

~S1606                    ~S1605
MEASURE FIRST        STORE USER'S BODY
EXERCISE QUANTITY    INFORMATION AND EXERCISE
INFORMATION          GOAL INFORMATION

                                              S1610
TRANSMIT EXERCISE      S1607
QUANTITY INFORMATION                    USER CERTIFICATION

                     ~S1608  S1611
STORE FIRST EXERCISE
QUANTITY INFORMATION           MEASURE SECOND
                               EXERCISE QUANTITY
~S1609                         INFORMATION
GENERATE FIRST HEALTH
MANAGEMENT DATA

TRANSMIT SECOND EXERCISE
QUANTITY INFORMATION

~S1613
STORE SECOND EXERCISE
QUANTITY INFORMATION     S1612

~S1614
GENERATE SECOND HEALTH
MANAGEMENT DATA

REQUEST FOR
S1615 HEALTH MANAGEMENT DATA

TRANSMIT SECOND HEALTH
MANAGEMENT DATA

~S1617
GENERATE HEALTH        S1616
MANAGEMENT UI

~S1618
DISPLAY HEALTH
MANAGEMET UI

32

Europäisches
Patentamt

European
Patent Office

Office européen
des brevets

# EUROPEAN SEARCH REPORT

Application Number

EP 12 19 1069

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP 2000 070242 A (MITSUBISHI ELECTRIC CORP) 7 March 2000 (2000-03-07) <br> * figure 1 * <br> * paragraphs [0025], [0026], [0030] * | 1-15 | INV. <br> A61B5/11 <br> A61B5/22 <br> A63B24/00 |
| X | JP 2007 143748 A (SHARP KK) 14 June 2007 (2007-06-14) <br> * figure 1 * <br> * paragraphs [0012] - [0016], [0039], [0049] * | 1-15 | |
| X | WO 02/43352 A2 (CLEVER SYS INC [US]) 30 May 2002 (2002-05-30) <br> * figure 1 * <br> * page 4, line 4 - line 13 * <br> * page 5, line 10 - line 16 * <br> * page 16, line 29 - page 17, line 12 * <br> * page 17, line 21 - line 23 * <br> * page 21, line 31 - page 22, line 6 * | 1,2,10, 11 | |
| A | WO 2008/081553 A1 (FUJITSU LTD [JP]; KASAMA KOUICHIROU [JP]; KANNO HIROSHI [JP]) 10 July 2008 (2008-07-10) <br> * paragraphs [0036], [0037], [0039] * | 3,12 | TECHNICAL FIELDS SEARCHED (IPC) <br><br> A61B <br> A63B |
| A | WO 2009/123396 A2 (KOREA ELECTRONICS TELECOMM [KR]; KIM SUNG-EUN [KR]; KANG SUNG-WEON [KR] 8 October 2009 (2009-10-08) <br> * paragraph [0030] * | 9 | |

The present search report has been drawn up for all claims

| Place of search | Date of completion of the search | Examiner |
|---|---|---|
| The Hague | 24 April 2013 | Vanderperren, Yves |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 12 19 1069

This annex lists the patent family members relating to the patent documents cited in the above-mentioned European search report.
The members are as contained in the European Patent Office EDP file on
The European Patent Office is in no way liable for these particulars which are merely given for the purpose of information.

24-04-2013

| Patent document cited in search report | | Publication date | Patent family member(s) | | Publication date |
|---|---|---|---|---|---|
| JP 2000070242 | A | 07-03-2000 | JP | 4021066 B2 | 12-12-2007 |
| | | | JP | 2000070242 A | 07-03-2000 |
| JP 2007143748 | A | 14-06-2007 | NONE | | |
| WO 0243352 | A2 | 30-05-2002 | AU | 3927202 A | 03-06-2002 |
| | | | EP | 1337962 A2 | 27-08-2003 |
| | | | JP | 2004514975 A | 20-05-2004 |
| | | | US | 6678413 B1 | 13-01-2004 |
| | | | US | 2004131254 A1 | 08-07-2004 |
| | | | US | 2004141635 A1 | 22-07-2004 |
| | | | US | 2004141636 A1 | 22-07-2004 |
| | | | US | 2007175406 A1 | 02-08-2007 |
| | | | US | 2007229522 A1 | 04-10-2007 |
| | | | US | 2009285452 A1 | 19-11-2009 |
| | | | US | 2009296992 A1 | 03-12-2009 |
| | | | US | 2011007946 A1 | 13-01-2011 |
| | | | WO | 0243352 A2 | 30-05-2002 |
| WO 2008081553 | A1 | 10-07-2008 | JP | 4952721 B2 | 13-06-2012 |
| | | | WO | 2008081553 A1 | 10-07-2008 |
| WO 2009123396 | A2 | 08-10-2009 | CN | 101983090 A | 02-03-2011 |
| | | | EP | 2262573 A2 | 22-12-2010 |
| | | | JP | 2011516915 A | 26-05-2011 |
| | | | US | 2011006926 A1 | 13-01-2011 |
| | | | WO | 2009123396 A2 | 08-10-2009 |

EPO FORM P0459

For more details about this annex : see Official Journal of the European Patent Office, No. 12/82